# EUROPEAN PATENT SPECIFICATION

(11) **EP 2 571 485 B1**
(45) Date of publication and mention of the grant of the patent: **16.02.2022**
(21) Application number: 11743378.9
(22) Date of filing: 16.05.2011
(51) Int. Cl.: A61K 9/46, A61K 31/137, A61K 31/435, A61K 31/4465

(54) **EFFERVESCENT ANTIHISTAMINE FORMULATIONS**
ANTIHISTAMIN-BRAUSEFORMULIERUNGEN
FORMULATIONS ANTIHISTAMINIQUES EFFERVESCENTES

(30) Priority: 18.05.2010 TR 201003942
(43) Date of publication of application: 27.03.2013
(73) Proprietor: Bilgic, Mahmut, 34220 Esenler-Istanbul (TR)
(72) Inventor: Bilgic, Mahmut, 34220 Esenler-Istanbul (TR)
(86) International application number: PCT/TR2011/000108
(87) International publication number: WO 2011/146030

(56) References cited:
- WO-A1-2006/069213
- WO-A2-2008/005267
- US-A1- 2003 216 423
- US-A1- 2004 057 995
- US-B1- 6 649 186
- Anonymous: "Remington: The Science and Practice of Pharmacy", 2000, Lippincott Williams & Wilkins, XP002667648, page 865

## Description

The present invention relates to effervescent formulations comprising desloratadine or pharmaceutically acceptable salt, solvate, derivative polymorph, hydrate or enantiomer thereof in a therapeutically effective amount as the active agent in order to be used in production of a medicament for elimination, prophylaxis and/or treatment of seasonal allergic rhinitis, perennial allergic rhinitis, chronic idiopathic urticaria and symptoms thereof.

### Background of the Invention

Desloratadine is a non-sedating, long-acting H1-antihistaminic which was first disclosed in the patent numbered US4659716A and has the chemical name 8-chloro-6,11-dihydro-11-(4-piperdinylidene)- 5*H*-benzo[5,6]cyclohepta[1,2-b]pyridine (Formula I).

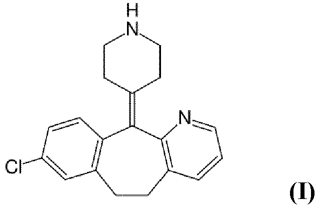

Desloratadine which is an active metabolite of loratadine is an HI receptor antagonist used by the oral route. Desloratadine is a new antihistaminic that binds to histamine HI receptors with higher affinity than HI receptor antagonists. Desloratadine competes with histamine and prevents histamine to bind to HI receptors. This antagonism blocks the effects of histamine on gastrointestinal channel, uterus, large blood vessels and bronchial smooth muscle. Blockage of HI receptors impedes histaminic activities such as edema, warmness and itchiness.

Desloratadine has an antiallergic and anti-inflammatory activity. The studies conducted have presented that desloratadine inhibits the wide range of chain of events starting and spreading out the allergic inflammation.

Desloratadine preparations on the market are in tablet, syrup and orally disintegrating tablet form. As it is known, solid dosage forms such as tablets have lower bioavailability than solution forms and they generally pose difficulty of use for geriatric, pediatric and physically handicapped patients who have swallowing difficulties. Oral solution and suspension forms produced as an alternative to these forms, on the other hand, are not preferable much as the stability of active agents cannot be maintained for long and they have short shelf life. Another disadvantage of these forms is that they have a risk of high and/or uncontrolled dose intake in pediatric and geriatric patients.

In addition, desloratadine poses problems in course of use and manufacture due to problems of stability, unpleasant taste and compatibility it has. For instance, pharmaceutically acceptable excipients, especially the ones having acidic characteristics, used in the production of tablet formulations causes the formulations to disintegrate fast and leads to a decrease in the stability of formulations since they are incompatible with desloratadine.

Syrup formulations, on the other hand, are not preferred due to the unpleasant taste of the active agent and the failure to maintain the stability of the solution.

Desloratadine's incompatibility with pharmaceutically acceptable excipients and low stability causes restrictions in formulations. Some examples of the substances that are incompatible with desloratadine are stearic acid, povidone, crospovidone, silicon dioxide, lactose, lactose monohydrate, sodium benzoate and glyceryl behenate. Desloratadine reacts with these excipients that are compulsory to use particularly in tablet formulations, leads to production of N-formyl derivatives and disintegration of the formulation.

In this respect, the same problem is experienced in desloratadine combinations, too. In combination products comprising a second active agent in addition to desloratadine, it can only be possible to solve individual problems of the two active agents in the same formulation by means of selecting pharmaceutical excipients that are compatible with the active agents and the other excipients.

There exist some propositions to solve these problems in the prior art. For instance, the patent numbered US6100274 A discloses pharmaceutical formulations comprising a basic salt in a sufficient amount to protect desloratadine against disintegration and at least one disintegrant. As another example, the patent numbered EP1728513 A2 discloses stable pharmaceutical compositions comprising desloratadine in combination with at least one amino acid which is in a sufficient amount to stabilize desloratadine.

As a further example, the patent numbered US 2003/216423 A1 relates to a stable pharmaceutical composition comprising loratadine and a nasal decongestant. WO 2006/069213 A1 relates to storage stable desloratadine syrup compositions.

However, these solutions remain incapable in solving incompatibility problem of desloratadine with active agents. In this respect, there is need for new and enhanced formulations and new dosage forms of desloratadine.

The inventors have produced new and enhanced effervescent formulations of desloratadine in order to solve the problems specified hereinabove.

A specific method and formulation have been used so as to produce the effervescent formulations of the present invention. In this way, not only stability and incompatibility with pharmaceutical excipients of desloratadine are solved but also user-friendly, reliable, new pharmaceutical formulations with high bioavailability which appeal to a wide range of patient profiles have been prepared.

The term "desloratadine" used throughout the text refers to desloratadine or a pharmaceutically acceptable salt, solvate, derivative, polymorph, hydrate or enantiomers thereof.

### Detailed Explanation of the Invention

The present invention relates to obtainment of new stable formulations comprising a therapeutically effective amount of desloratadine in which the bitter taste is masked.

As explained before, it is quite difficult to formulate desloratadine. It causes side reaction with excipients that are frequently used in pharmaceutical technology and leads to limitations in excipient selection.

Moreover, it is not preferred in syrup and chewable tablet form by patients due to the bitter taste it has.

As a result of the studies they conducted, the inventors have surprisingly produced stable, user-friendly effervescent formulations which have high bioavailability and long shelf life by means of selecting effective and sufficient amount of pharmaceutical excipients and suitable manufacturing method. It has been observed that effervescent desloratadine formulations produced according to the present invention do not present any incompatibility with pharmaceutical excipients and hence did not lead to any decrease in the stability.

The effervescent formulations of the present invention do not require an extra preservative agent or stabilizer addition since they are consumed shortly after they are prepared. This is a significant advantage for desloratadine formulations having compatibility problems with pharmaceutical excipients.

As the formulations of the present invention are in effervescent form, they have higher bioavailability than solid dosage forms. Furthermore, these forms appeal to a wider range of patient profile in terms of ease of use and carrying.

The effervescent formulations of the present invention can be formulated with a second active agent. Nasal decongestants, leukotriene receptor antagonists, antihistaminics, antidepressants can be used as the second active agent. Preferred nasal decongestant used in the formulations according to the invention is pseudoephedrine.

The formulations of the present invention are used in production of a medicament for elimination, prophylaxis and/or treatment of seasonal allergic rhinitis, perennial allergic rhinitis, chronic idiopathic urticaria and symptoms thereof.

The effervescent formulations of the present invention comprises effective amounts of desloratadine, at least one pharmaceutically acceptable acid-base couple, at least one filling material, at least one binder, at least one sweetener, at least one flavoring agent and at least one more pharmaceutical excipient.

The sweeteners which can be used in the effervescent formulations of the present invention can be selected from a group comprising synthetic and natural sugars; sucrose, sucralose, saccharine, aspartame, acesulfame, thaumatin, dextrose, mannitol, lactose, xylitol, isomalt, isomaltol, lactitol, eryhtrol, maltodextrin, alpha, beta and gamma cyclodextrins, dihydrochalcone, alitam, sorbitol, sodium cyclamate, miraculin, monellin, steviocide and/or pharmaceutically acceptable salts thereof.

The effervescent acids which can be used in the effervescent formulations of the present invention can be selected from a group comprising citric acid, monosodium citrate, tartaric acid, fumaric acid, malic acid and/or their pharmaceutically acceptable salts, hydrates, anhydrates or preferably a mixture thereof.

The effervescent bases which can be used in the effervescent formulations of the present invention can be selected from a group comprising sodium, potassium and calcium carbonates, bicarbonates and/or sodium glycine carbonate or a combination thereof. The effervescent couple used in the formulations of the present invention is preferably composed of hydrogen carbonate and citric acid.

The filling materials which can be used in the effervescent formulations of the present invention can be selected from a group comprising talc, calcium carbonate, microcrystalline cellulose, powderized cellulose, dextrates, kaolin, mannitol, silicic acid, sorbitol, starch, pregelatinized starch, maltodextrin or a combination thereof. The filling material used in the formulations of the present invention is preferably maltodextrin.

The binders which can be used in the effervescent formulations of the present invention can be selected from a group comprising substances selected from the group comprising potato starch, wheat starch or corn starch; microcrystalline cellulose, hydroxypropyl cellulose, sorbitol, hydroxyethyl cellulose; hydroxypropylmethyl cellulose, for instance hydroxypropylmethyl cellulose- Type 2910 USP; hypromellose and polyvinylpyrrolidone or mixtures thereof. Preferably, sorbitol is used as the binder in the effervescent formulations of the present invention.

The flavoring agents which can be used in the effervescent formulation of the present invention can be banana, strawberry, lemon, orange, peach, vanilla, blackberry or a similar natural fruit or an aromatic plant flavor. The flavoring agent used in the effervescent formulations of the present invention is preferably blackberry flavor.

The characteristic feature of the formulations of the present invention is that said formulations comprise;
a) desloratadine in the range of 0.1% to 10 by weight,
b) an effervescent acid-base couple in the range of 50% to 90% by weight,
c) at least one filling material in the range of 1% to 20% by weight,
d) at least one binder in the range of 1% to 10% by weight,
e) at least one sweetener in the range of 0.1% to 10% by weight,
f) at least one flavoring agent in the range of 0.1% to 5% by weight and preferably
g) at least one more pharmaceutical excipient in the range of 0.1% to 20% by weight.

The effervescent formulations of the present invention can be produced by methods known in the prior art. However, disclosed herein is also a production method for the effervescent formulations of the present invention involving wet granulation.

The proposed production method basically comprises the following steps:
1. Preparation of granulation solution; characterized in that 10%-40% of total amount of the filling material and 10%-30% of total amount of the binder by weight, are used for preparation of the granulation solution
2. Preparation of active agent mixture
3. Wet granulation
4. Sieving and tablet compression

The granulation solution to be used for producing the effervescent formulations of the present invention given above comprises deionized water in an effective amount, at least one pharmaceutically acceptable binder and filling material. However, the inventors encountered some problems in the case that all of the binder and the filling material was used for the granulation solution. Addition of these two excipients in high amounts resulted in gelling while addition of them in low amounts leaded to failure in sufficient granulation.

The inventors specifically determined the amount of the binder and the filling material comprised in the granulation solution as given below and the granules of the active agent were obtained as desired this way.

In the first step of the production method, purified water, some part of the pharmaceutically acceptable filling material and the binder comprised in the formulation are mixed in order to prepare the granulation solution.

10%-40% of total amount of the filling material and 10%-30% of total amount of the binder by weight are used for preparation of the granulation solution. Final content of the granulation solution prepared according to this is as follows:
- Purified water in an amount in the range of 10% to 90% by weight
- At least one pharmaceutically acceptable binder in an amount in the range of 1% to 40% by weight
- At least one pharmaceutically acceptable filling material in the range of 1% to 50% by weight.

The percentage of water in the granulation solution used in production of the formulations is in the range of 10% to 90% by weight, preferably in the range of 10% to 80% by weight, more preferably %10 to 70; the amount of the binder is in the range of 1% to 40% by weight, preferably in the range of 10% to 40% by weight, more preferably 10% to 30%; the amount of the filling material is in the range of 1% to 50% by weight, preferably in the range of 5% to 40% by weight, more preferably 10% to 40% .

In the second step of the production method, an effective amount of desloratadine, a pharmaceutically acceptable effervescent acid-base couple, at least one sweetener and the rest of the pharmaceutically acceptable binder and the filling material some of which is used in the first step are mixed.

In the third step of the production method, the active agent mixture is wet granulated with the granulation solution prepared as described in the first step. The granules obtained are sieved and tablets are compressed of this granules.

In this way, the active agent mixture which was granulated with the granulation solution prepared did not present gelling or effervescent reaction during production and thus, granules having sufficient granule size, moisture rate and stability were obtained.

### EXAMPLES

Example 1. Effervescent formulation comprising desloratadine

| **Content** | **Percentage (%) of Weight** |
|---|---|
| Desloratadine | 0.30 |
| Effervescent couple | 80.00 |
| Filling material | 10.00 |
| Binder | 3.00 |
| At least one sweetener | 3.20 |
| Flavoring agent | 3.50 |
| **Total** | **100** |

## Claims

1. A pharmaceutical formulation comprising desloratadine in an effective amount **characterized in that** said formulation is in effervescent form.

2. The effervescent formulation according to claim 1 **characterized in that** said formulation is composed of at least one pharmaceutically acceptable binder, at least one filling material, at least one effervescent acid-base couple, at least one sweetener and at least one more pharmaceutical excipient.

3. The effervescent formulation according to claims 1 to 2 **characterized in that** said formulations comprise;
• desloratadine in the range of 0.1 % to 10 by weight,
• an effervescent acid-base couple in the range of 50% to 90% by weight,
• at least one filling material in the range of 1% to 20% by weight,
• at least one binder in the range of 1 % to 10% by weight,
• at least one sweetener in the range of 0.1 % to 10% by weight,
• at least one flavoring agent in the range of 0.1% to 5% by weight and preferably
• at least one more pharmaceutical excipient in the range of 0.1% to 20% by weight.

4. The effervescent formulation according to claims 1 to 3 **characterized in that** the effervescent acid-base couple is selected from a group comprising potassium carbonate, potassium bicarbonate, potassium citrate, potassium hydroxide, sodium carbonate, sodium bicarbonate, monosodium citrate, citric acid, lactic acid, malic acid, tartaric acid or combinations thereof.

5. The effervescent acid-base couple according to claim 4, wherein said effervescent acid-base couple is preferably citric acid and sodium bicarbonate.

6. The effervescent formulation according to claim 2 **characterized in that** the filling material is selected from a group comprising talc, calcium carbonate, microcrystalline cellulose,powderized cellulose, dextrates, kaolin, mannitol, silicic acid, sorbitol, starch, pregelatinized starch, maltodextrin or a combination thereof.

7. The filling material according to claim 6, wherein said filling material is maltodextrin.

8. The effervescent formulation according to claim 2 **characterized in that** the pharmaceutically acceptable binder is selected from a group comprising potato starch, wheat starch or corn starch; microcrystalline cellulose hydroxypropyl cellulose, sorbitol, hydroxyethyl cellulose; hydroxypropylmethyl cellulose, hypromellose and polyvinypyrrolidone or a combination thereof.

9. The binder according to claim 8, wherein said binder is sorbitol.

10. The effervescent formulation according to claim 2 **characterized in that** the pharmaceutically acceptable sweetener is selected from a group comprising synthetic and natural sugars; sucrose, sucralose, saccharine, aspartame, acesulfame, thaumatin, dextrose, mannitol, lactose, xylitol, isomalt, isomaltol, lactitol, eryhtrol, maltodextrin, alpha, beta and gamma cyclodextrins, dihydrochalcone, alitam, sorbitol, sodium cyclamate, miraculin, monellin, steviocide and/or pharmaceutically acceptable salts or combinations thereof.

11. The sweetener according to claim 10, wherein said sweetener is acesulfame and acesulfame potassium.

12. The effervescent formulation according to claim 2 **characterized in that** the pharmaceutically acceptable flavoring agent is selected from a group comprising banana, strawberry, lemon, orange, peach, vanilla, blackberry or a similar natural fruit or an aromatic plant flavor.

13. The flavoring agent according to claim 12, wherein said flavoring agent is blackberry flavor.

14. A production method for an effervescent formulation according to any preceding claims, wherein said method comprises the following steps respectively:
a) Preparation of granulation solution; **characterized in that** 10%-40% of total amount of the filling material and 10%-30% of total amount of the binder by weight, are used for preparation of the granulation solution
b) Preparation of active agent mixture
c) Wet granulation
d) Sieving and tablet compression

## Patentansprüche

1. Pharmazeutische Formulierung, umfassend Desloratadin in einer wirksamen Menge, **dadurch gekennzeichnet, dass** die genannte Formulierung in Brauseform ist.

2. Brauseformulierung nach Anspruch 1, **dadurch gekennzeichnet, dass** die genannte Formulierung aus mindestens einem pharmazeutisch akzeptablen Bindemittel, mindestens einem Füllmaterial, mindestens einem brausenden Säure-Basen-Paar, mindestens einem Süßstoff und mindestens einem weiteren pharmazeutischen Exzipienten besteht.

3. Brauseformulierung nach einem der Ansprüche 1 bis 2, **dadurch gekennzeichnet, dass** die genannte Formulierung umfasst:
• Desloratadin im Bereich von 0.1 bis 10 Gew.-%,
• ein brausendes Säure-Basen-Paar im Bereich von 50 bis 90 Gew.-%,
• mindestens einen Füllmaterial im Bereich von 1 bis 20 Gew.-%,
• mindestens ein Bindemittel im Bereich von 1 bis 10 Gew.-%,
• mindestens einen Süßstoff im Bereich von 0.1 bis 10 Gew.-%,
• mindestens einen Geschmacksstoff im Bereich von 0.1 bis 5 Gew.-% und vorzugsweise
• mindestens einen weiteren pharmazeutischen Exzipienten im Bereich von 0.1 bis 20 Gew.-%.

4. Brauseformulierung nach einem der Ansprüche 1 bis 3, **dadurch gekennzeichnet, dass** das brausende Säure-Basen-Paar aus einer Gruppe ausgewählt ist, die Kaliumcarbonat, Kaliumbicarbonat, Kaliumcitrat, Kaliumhydroxid, Natriumcarbonat, Natriumbicarbonat, Mononatriumcitrat, Zitronensäure, Milchsäure, Äpfelsäure, Weinsäure oder Kombinationen davon umfasst.

5. Brause-Säure-Basen-Paar nach Anspruch 4, wobei das genannte Brause-Säure-Basen-Paar vorzugsweise Zitronensäure und Natriumbicarbonat ist.

6. Brauseformulierung nach Anspruch 2, **dadurch gekennzeichnet, dass** das Füllmaterial aus einer Gruppe ausgewählt ist, die Talk, Calciumcarbonat, mikrokristalline Cellulose, pulverisierte Cellulose, Dextrate, Kaolin, Mannit, Kieselsäure, Sorbit, Stärke, vorgelatinierte Stärke, Maltodextrin oder eine Kombination davon umfasst.

7. Füllmaterial nach Anspruch 6, wobei das genannte Füllmaterial Maltodextrin ist.

8. Brauseformulierung nach Anspruch 2, **dadurch gekennzeichnet, dass** das pharmazeutisch verträgliche Bindemittel aus einer Gruppe ausgewählt ist, die Kartoffel-, Weizen- oder Maisstärke, mikrokristalline Cellulose, Hydroxypropylcellulose, Sorbit, Hydroxyethylcellulose, Hydroxypropylmethylcellulose, Hypromellose und Polyvinypyrrolidon oder eine Kombination davon umfasst.

9. Bindemittel nach Anspruch 8, wobei das genannte Bindemittel Sorbitol ist.

10. Brauseformulierung nach Anspruch 2, **dadurch gekennzeichnet, dass** der pharmazeutisch akzeptable Süßstoff aus einer Gruppe ausgewählt ist, die synthetische und natürliche Zucker umfasst; Saccharose, Saccharin, Aspartam, Acesulfam, Thaumatin, Dextrose, Mannit, Lactose, Xylit, Isomalt, Isomaltol, Lactitol, Eryhtrol, Maltodextrin, Alpha-, Beta- und Gamma-Cyclodextrine, Dihydrochalkon, Alitam, Sorbit, Natriumcyclamat, Miraculin, Monellin, Steviocid und/oder pharmazeutisch akzeptable Salze oder Kombinationen davon.

11. Süßstoff nach Anspruch 10, wobei der genannte Süßstoff Acesulfam und Acesulfamkalium ist.

12. Brauseformulierung nach Anspruch 2, **dadurch gekennzeichnet, dass** der pharmazeutisch verträgliche Geschmacksstoff aus einer Gruppe ausgewählt ist, die Banane, Erdbeere, Zitrone, Orange, Pfirsich, Vanille, Brombeere oder einen ähnlichen natürlichen Frucht- oder aromatischen Pflanzengeschmack umfasst.

13. Geschmacksstoff nach Anspruch 12, wobei der genannte Geschmacksstoff Brombeer-Aroma ist.

14. Verfahren zur Herstellung einer Brauseformulierung nach einem der vorhergehenden Ansprüche, wobei das genannte Verfahren jeweils die folgenden Schritte umfasst:
a) Bereitstellen der Granulationslösung, **dadurch gekennzeichnet, dass** 10 bis 40 Gew.-% der Gesamtmenge des Füllmaterials und 10 bis 30 Gew.-% der Gesamtmenge des Bindemittels für die Herstellung der Granulationslösung verwendet werden
b) Bereitstellen der Wirkstoffmischung
c) Nassgranulation
d) Sieben und Verpressen der Tabletten

## Revendications

1. Formulation pharmaceutique comprenant la desloratadine en une quantité efficace, **caractérisée en ce que** ladite formulation est sous forme effervescente.

2. Formulation effervescente selon la revendication 1, **caractérisée** en cc que ladite formulation est composée d'au moins un liant pharmaceutiquement acceptable, d'au moins un matériau de remplissage, d'au moins un couple acide-base effervescent, d'au moins un édulcorant et d'au moins un excipient pharmaceutique supplémentaire.

3. Formulation effervescente selon les revendications 1 ou 2, **caractérisée en ce que** lesdites formulations comprennent;
• la desloratadine dans la gamme de 0.1 % à 10% en poids,
• un couple acide-base effervescent dans la gamme de 50% à 90% en poids,
• au moins un matériau de remplissage dans la gamme de 1% à 20% en poids,
• au moins un liant dans la gamme de 1% à 10% en poids,
• au moins un édulcorant dans la gamme de 0.1% à 10% en poids,
• au moins un agent aromatisant dans la gamme de 0.1% à 5% en poids et de préférence
• au moins un autre excipient pharmaceutique dans la gamme de 0.1% à 20% en poids.

4. Formulation effervescente selon les revendications 1 ou 3, **caractérisée en ce que** le couple acide-base effervescent est choisi dans un groupe comprenant le carbonate de potassium, le bicarbonate de potassium, le citrate de potassium, l'hydroxyde de potassium, le carbonate de sodium, le bicarbonate de sodium, le citrate monosodique, l'acide citrique, l'acide lactique, l'acide malique, l'acide tartrique ou leurs combinaisons.

5. Couple acide-base effervescent selon la revendication 4, dans lequel ledit couple acide-base effervescent est de préférence l'acide citrique et le bicarbonate de sodium.

6. Formulation effervescente selon la revendication 2, **caractérisée en ce que** le matériau de remplissage est choisi dans un groupe comprenant le talc, le carbonate de calcium, la cellulose microcristalline, la cellulose en poudre, les dextrates, le kaolin, le mannitol, l'acide silicique, le sorbitol, l'amidon, l'amidon prégélatinisé, la maltodextrine ou une combinaison de ceux-ci.

7. Matériau de remplissage selon la revendication 6, dans lequel ledit matériau de remplissage est la maltodextrine.

8. Formulation effervescente selon la revendication 2, **caractérisée en ce que** le liant pharmaceutiquement acceptable est choisi dans un groupe comprenant l'amidon de pomme de terre, l'amidon de blé ou l'amidon de maïs ; la cellulose microcristalline ; l'hydroxypropylcellulose, le sorbitol, l'hydroxyéthylcellulose ; l'hydroxypropylméthylcellulose, l'hypromellose et la polyvinypyrrolidone ou une combinaison de ceux-ci.

9. Liant selon la revendication 8, dans lequel ledit liant est le sorbitol.

10. Formulation effervescente selon la revendication 2, **caractérisée en ce que** l'édulcorant pharmaceutiquement acceptable est choisi dans un groupe comprenant les sucres synthétiques et naturels ; le saccharose, le sucralose, la saccharine, l'aspartame, l'acésulfame, la thaumatine, le dextrose, le mannitol, le lactose, le xylitol, l'isomalt, l'isomaltol, le lactitol, l'eryhtrol, la maltodextrine, les alpha, bêta et gamma cyclodextrines, la dihydrochalcone, l'alitam, le sorbitol, le cyclamate de sodium, la miraculine, la monelline, le stéviocide et/ou leurs sels ou combinaisons pharmaceutiquement acceptables.

11. Édulcorant selon la revendication 10, dans lequel ledit édulcorant est l'acésulfame et l'acésulfame de potassium.

12. Formulation effervescente selon la revendication 2, **caractérisée en ce que** l'agent aromatisant pharmaceutiquement acceptable est choisi dans un groupe comprenant la banane, la fraise, le citron, l'orange, la pêche, la vanille, la mûre ou un fruit naturel similaire ou un arôme de plante aromatisante.

13. Agent aromatisant selon la revendication 12, dans lequel ledit agent aromatisant est un arôme de mûre.

14. Procédé de production de formulation effervescente selon l'une quelconque des revendications précédentes, dans lequel ledit procédé comprend les étapes suivantes respectivement :
a) Préparation de la solution de granulation ; **caractérisé en ce que** 10% - 40% de la quantité totale du matériau de remplissage et 10% - 30% de la quantité totale du liant, en poids, sont utilisés pour la préparation de la solution de granulation
b) Préparation du mélange d'agents actifs
c) Granulation humide
d) Tamisage et compression des comprimés
